# EUROPÄISCHE PATENTANMELDUNG

(11) **EP 2 609 829 A2**
(43) Veröffentlichungstag der Anmeldung: **03.07.2013**
(21) Anmeldenummer: 12187486.1
(22) Anmeldetag: 05.10.2012
(51) Int. Cl.: A45D 2/00, A45D 7/00, A61Q 5/06, A61Q 5/12, A61K 8/92

(54) **Haarstyling- und/oder Haarglättmittel mit zugkraftverstärkendem Mittel**

(30) Priorität: 10.10.2011 DE 102011084188
(71) Anmelder: BSH Bosch und Siemens Hausgeräte GmbH, 81739 München (DE)
(72) Erfinder: Altmann, Berthold, 83374 Oderberg (DE); Blischke, Daniela, 92360 Mühlhausen (DE); Copitzky, Thomas, 83278 Traunstein (DE)

(57) **Zusammenfassung**

Die Erfindung betrifft ein Haarstyling- und/oder Haarpflegemittel für die Aufbringung auf das Haar und zur Anwendung im Zusammenwirken mit einem beheizbaren Haarglätter, mit einem bei der Haarglättung zugkraftverstärkenden Mittel sowie die Verwendung dieses Mittels mit einem beheizbaren Haarglätter.

## Beschreibung

Die Erfindung betrifft ein Haarstyling- und/oder Haarpflegemittel für die Aufbringung auf das Haar und zur Anwendung im Zusammenwirken mit einem beheizbaren Haarglätter sowie dessen Verwendung.

Ein für einen Haarstylingprozess üblicherweise verwendeter Haarglätter ist ein Gerät, das z.B. zwei gegenüberliegende beheizbare Glättplatten umfasst. Diese können ähnlich einer Zange mittels einer Handbewegung zusammengedrückt werden, so dass damit eine Haarsträhne aufgenommen werden kann. Die beiden Glättplatten sind mittels eingebauter Heizvorrichtungen beheizbar ausgestaltet, so dass damit innerhalb einer kurzen Zeitspanne von wenigen Zehntelsekunden bis zu wenigen Sekunden die Wärme auf die zwischen den beiden Glättplatten liegenden Haare übertragen werden kann.

Bei einem Stylingprozess wird eine Haarsträhne oder vereinzelte bzw. mehrere Haare zwischen den beiden Glättplatten aufgenommen, durch Zusammendrücken der beiden Glättplatten festgehalten und durch Einrollen oder Glattziehen der Haare meist unter Zug in die gewünschte Form gebracht. In die gewünschte Form bringen bedeutet nicht nur das Lockenwickeln, sondern insbesondere auch das Glätten der Haare unter Erhitzen und gleichzeitigem Aufbringen einer Zugkraft auf die Haare.

Die Glättplatten von herkömmlichen Haarglättern sind zum Beispiel aus Alu-Stranggussprofilen, Keramiklegierungen, eloxierten Legierungen, Emaillegierungen. In der EP 1 698 249 A1 ist ein Haarglätter mit einer ersten und einer zweiten beheizbaren Glättplatte und einem auf mindestens einer der Glättplatten vorgesehenem Pufferelement aus einem wärmebeständigen Silicon, Silber, Teflon oder einem Urethankautschuk beschrieben. Das auf oder in den Glättplatten angeordnete Pufferelement soll ein Ausreißen der Haare vermeiden und eine Verstärkung des Einroll- bzw. des Glätteffekts bewirken.

Neben modifizierten Glättplatten gibt es Hilfsmittel bzw. Pflegemittel, die im Zusammenhang mit der Haarglättung vor dem Glättprozess auf das Haar aufgebracht werden. Hierzu zählen zum Beispiel Hitzeschutzsprays, die zumeist aus leicht flüchtigen Substanzen bestehen und das Haar dadurch während des Glättens kühlen. Dabei besteht dann aber die Gefahr, dass die Haare keine ausreichende Wärmezufuhr für einen hinreichenden Glättprozess erfahren oder dass der Anwender zur Umgehung dieses Effekts eine höhere Temperatur einstellt.

Aufgabe der Erfindung ist es, alternative Mittel bereitzustellen, mit denen mechanische Haarschädigungen während des Haarstyling- und/oder Haarpflegeprozesses reduziert werden.

Diese Aufgabe wird durch ein Haarstyling- und/oder Haarpflegemittel gemäß Oberbegriff mit den in Anspruch 1 angegebenen Merkmalen gelöst. Weiterhin wird die Aufgabe durch eine Verwendung nach Anspruch 9 gelöst.

Das erfindungsgemäße Haarstyling- und/oder Haarpflegemittel für die Aufbringung auf das Haar und zur Anwendung im Zusammenwirken mit einem beheizbaren Haarglätter umfasst ein bei der Haarglättung zugkraftverstärkendes Mittel. Das erfindungsgemäße Haarstyling- und/oder Haarpflegemittel (der Einfachheit halber im Folgenden nur "Mittel" genannt) ist dabei ein Mittel, mit dem Haare in Form gebracht werden können, insbesondere geglättet werden können, und/oder bei der Haarglättung gepflegt werden können. Dabei fällt unter ein Pflegemittel auch ein Mittel, das die Haare während des Glättprozesses schont, z.B. vor mechanischen Beschädigungen schützt.

Ein bei der Haarglättung zugkraftverstärkendes Mittel ist ein Mittel, das die Zugkraft, die auf die Haarsträhne bei der Anwendung eines Haarglätters wirkt, im Vergleich zu einer Anwendung ohne ein solches Mittel vergrößert. Eine Erhöhung der Zugkraft kann zum Beispiel durch eine Erhöhung der Reibung zwischen Plattenoberfläche und Haaroberfläche bewerkstelligt werden. Dadurch kann man bei gleicher Temperatur der Glättplatten zum Beispiel den notwendigen Anpressdruck auf die Haarsträhne bei gleich bleibendem Glättergebnis verringern. Dies bewirkt gleichzeitig, dass eine geringere mechanische Belastung auf das Haar ausgeübt wird und somit auch eine mechanische Beschädigung reduziert oder verhindert werden können.

Die erfindungsgemäße Verwendung umfasst die Anwendung eines erfindungsgemäßen Haarstyling- und/oder Haarpflegemittels mit einem beheizbaren Haarglätter. In einem Haarstylingprozess wie bei der Haarglättung wird das erfindungsgemäße Mittel dabei vor der Anwendung des Haarglätters direkt auf die Haare aufgebracht. Das kann beispielsweise durch Aufsprühen, Auftragen, Aufstäuben oder Einmassieren erfolgen. Das Mittel kann daher für jede Applikationsform entsprechend formuliert werden, z.B. in Form eines Sprays, Schampoos, einer Spülung oder als Pulver.

Das erfindungsgemäße Haarstyling- und/oder Haarpflegemittel kann nach einer bevorzugten Ausgestaltung ein zugkraftverstärkendes Mittel mit Bestandteilen umfassen, deren Moleküle beim bestimmungsgemäßen Gebrauch an der Haaroberfläche adsorbiert werden. Dazu bilden die für die Wirkung relevanten Bestandteile Wechselwirkungskräfte zur Haaroberfläche aus, um dadurch auf der Haaroberfläche gehalten zu werden. Die Wechselwirkungskräfte können physikalischer - z.B. van-der-Waals-Kräfte - und/oder chemischer Natur - z.B. Ausbildung fester chemischer Bindungen - sein. Über die Wechselwirkungskräfte werden die für den Zugkrafterhöhungseffekt verantwortlichen Verbindungen auf der Haaroberfläche gehalten.

Nach einer weiteren bevorzugten Ausgestaltung oder einer Weiterbildung des erfindungsgemäßen Mittels kann das Mittel sich im bestimmungsgemäßen Gebrauch an der Haaroberfläche anlagernde Partikel umfassen. Sich an die Haaroberfläche anlagernde Partikel können beispielsweise in einem Medium, bevorzugt einem flüssigen Medium, dispergiert sein oder auch trocken, z.B. in Form eines Puders oder Pulvers vorliegen. Je nach Trägermedium können die Partikel auf die Haare aufgebracht werden. Beispiele hierfür sind ein Aufsprühen von in einer Lösung dispergierten Partikeln, das Auftragen bzw. Einmassieren der Partikel mittels einer Spülung, eines Gels oder einer Paste oder, wenn sie in Pulverform vorliegen, das Aufstäuben auf die Haare.

Auch die verwendeten Partikel werden dabei bevorzugt über Wechselwirkungskräfte auf der Haaroberfläche gehalten. Die Wechselwirkungskräfte können physikalischer - z.B. van-der-Waals-Kräfte - und/oder chemischer Natur - z.B. Ausbildung fester chemischer Bindungen - sein. Über diese Wechselwirkungskräfte werden die für den Zugkrafterhöhungseffekt verantwortlichen Partikel auf der Haaroberfläche gehalten.

Um eine erhöhte Reibung zwischen den Glättplatten und der Haaroberfläche zu erzielen, kann gemäß einer bevorzugten Ausführungsform das Haarstyling- und/oder Haarpflegemittel Spreit-Hilfsmittel umfassen, welches die Partikel oder Bestandteile nach dem Auftrag auf der Haaroberfläche verteilt. "Spreiten" ist die Verbreitung einer Substanz auf der Oberfläche einer anderen durch Wirkung der Grenzflächenspannung. Dadurch können molekulare Schichtstrukturen hergestellt werden. Beispiele von Spreit-Hilfsmitteln sind Lösungsmittel, Tenside, usw. Ein Spreit-Hilfsmittel ist dann nicht nötig, wenn die Partikel oder die wesentlichen Bestandteile des erfindungsgemäßen Mittels schon bei der bestimmungsgemäßen Anwendung von alleine teilweise oder vollständig Spreiten, so dass sie danach verteilt auf der Haaroberfläche vorliegen. Bevorzugt ist es, dass die an die Haaroberfläche anzulagernden Partikel oder Moleküle einen molekularen, zum Beispiel eine monomolekularen, Film auf der Haaroberfläche ausbilden. Wenn die Substanzmenge nicht für einen kompletten monomolekularen Film ausreicht, z. B. falls einzelne Partikel aufzubringen sind, ist es vorteilhaft, wenn sich entweder durch die Substanzeigenschaften alleine oder durch ein Spreit-Hilfsmittel, monomolekulare Gebiete ausbilden, die räumlich voneinander getrennt sind.

Als weitere Variante wäre es denkbar, dass die Partikel oder Bestandteile in mehreren Lagen, z.B. in zwei, drei, vier oder auch bis zu zehn oder mehr Lagen, oder als Cluster, d. h. in einer geordneten oder ungeordneten zusammenhängenden Struktur wie einer Haufenstruktur auf der Haaroberfläche angeordnet sind. Je nach Ausmaß der verwendeten Menge kann die gesamte Haaroberfläche mit derartigen Strukturen bedeckt sein oder es bilden sich räumlich voneinander getrennte Gebiete aus angelagerten Molekülen oder Partikeln aus.

Zur Ausbreitung der Partikel oder Moleküle auf der Haaroberfläche kann es auch bevorzugt sein, die aktiven Bestandteile des Mittels in einem oder mehreren Lösungsmitteln, die auch als Spreit-Hilfsmittel dienen können, aufzunehmen, zu lösen oder zu dispergieren. Gegebenenfalls können weitere Hilfsmittel wie z.B. Emulgatoren, die eine bessere Lösung der aktiven Bestandteile im Lösungsmittel erlauben, in dem Mittel umfasst sein. Aber auch ein Mittel, das komplett aus den aktiven Substanzen, z. B. den Partikeln, besteht, kann erfindungsgemäß eingesetzt werden, wenn durch die Auftragung auf die Haaroberfläche, z.B. durch ein Sprühen oder ein Aufstäuben, eine entsprechende Verteilung der aktiven Bestandteile auf der Haaroberfläche erfolgt.

Eine weitere bevorzugte Ausgestaltung des erfindungsgemäßen Haarstyling- und/oder Haarpflegemittels umfasst Elastomerpartikel als das zugkraftverstärkende Mittel. Elastomerpartikel sind in der Regel elastische Kunststoff- oder Kautschukpartikel, die bevorzugt bis zu etwa 150 bis 300 °C, weiter bevorzugt bis etwa 150 °C bis etwa 180 °C wärmestabil sind, d.h. sich nicht wesentlich zersetzen. Bei der Anwendung solcher Elastomerpartikel werden diese in der Regel mechanisch/plastisch verformbar (elastische Eigenschaft der Elastomere), wodurch es zu einer Erhöhung der Reibungskraft zwischen Haaroberfläche und Glättplatte kommt. Da diese Elastomerpartikel bevorzugt einer thermischen Zersetzung bei längerer Anwendung unterliegen können, ist eine weitgehend rückstandsfreie Anwendung möglich.

Einfache "handelsübliche" Elastomere wie Kautschuk oder Polyurethane (PUR) sind beispielhaft zu nennen. Im reinen Zustand kommen sie jedoch nur sehr eingeschränkt in Frage, da sie manchmal unvorteilhafte Zwischenzustände auf dem Weg zu ihrer Auflösung einnehmen. Ungewollte Beispiele hierfür wäre ein bei der Auflösung klebriger Kautschuk, eine zu hohe Temperatur, bei der eine weitestgehend vollständige Zersetzung stattfindet, oder Zersetzungsprodukte mit unvorteilhaften Eigenschaften, wie z. B. einem unangenehmen Geruch bei der Zersetzung auf Grund von z. B. schwefelhaltigen Elastomerformulierungen. Deswegen werden vorteilhafter Weise Elastomere eingesetzt, die sich auf Grund ihrer chemischen Struktur bei niedrigen Temperaturen, z. B. in einem Bereich von etwa 100 bis etwa 450 °C, weiter bevorzugt unterhalb von 200 °C zersetzten und/oder keine störenden Zersetzungsprodukte bilden. Alternativ können auch Hilfsmittel, wie z. B. Katalysatoren oder Reaktionspartner eingesetzt werden, mit deren Hilfe die Zersetzungstemperatur herabgesetzt werden kann und/oder die Bildung geeigneter, d.h. leicht flüchtiger und/oder unschädlicher, Zersetzungsprodukte erreicht werden kann. Ein Beispiel hierfür ist der Einsatz von Aluminiumchlorid für die Zersetzung von Kautschuk.

Die Größe der Partikel kann grundsätzlich über einen weiten Bereich variieren, denn die Größe und Form ist dabei eher von der Wirkweise abhängig als von rein geometrischen Überlegungen, solange sie eine zugkraftverstärkende Wirkung haben. Dabei ist die Partikelgröße im unteren molekularen Bereich bis hin zu einer oberen Grenze im Bereich eines oder einiger weniger Haardurchmessern. Um ein Verkleben der Haare in einer Haarsträhne zu verhindern, ist in einer weiteren bevorzugten Weiterbildung oder Ausführungsform des Haarstyling- und/oder Haarpflegemittels der Partikeldurchmesser der in dem Mittel als aktive Substanz enthaltenen Partikel kleiner oder gleich etwa 200 µm, bevorzugt kleiner oder gleich etwa 150 µm, weiter bevorzugt kleiner oder gleich 100 µm, insbesondere etwa 50 bis 100 µm.

Eine weitere bevorzugte Ausgestaltung des erfindungsgemäßen Haarstyling- und/oder Haarpflegemittels umfasst als das zugkraftverstärkende Mittel eine alkalische Komponente. Alkalische Verbindungen können auf der Haaroberfläche zu einer Erhöhung des pH-Wertes und/oder einer Quellung der Haare führen, so dass die Cuticula etwas absteht. Durch diesen Quelleffekt erzeugt das Mittel eine rauere Haaroberfläche, die eine verbesserte Reibungswirkung auf den Glättplatten bewirkt. Substanzen, die eine Quellung der Haare bewirken können, sind z. B. Natronlauge, Ammoniak oder Mercaptoverbindungen. Lösliche Silikate können ebenfalls eine Quellung der Haare verursachen, so dass sie im Rahmen der Erfindung als zugkraftverstärkende Mittel eingesetzt werden können.

Eine weitere bevorzugte Ausgestaltung des erfindungsgemäßen Haarstyling- und/oder Haarpflegemittels umfasst als das zugkraftverstärkende Mittel eine ölhaltige Komponente. Ölhaltige Komponenten auf der Haaroberfläche können zu einem Stick-Slip-Effekt führen, der in einer Erhöhung der Zugkraft beim Haareglätten resultieren kann.

In einer weiteren bevorzugten Ausführungsform umfasst das erfindungsgemäße Haarstyling- und/oder Haarpflegemittel ein zugkraftverstärkendes Mittel, welches eine Zugkraft zwischen Haaroberfläche und einer Glättplatte eines Haarglätters zwischen 2 bis 5 N, bevorzugt zwischen 2 bis 3 N bei der bestimmungsgemäßen Anwendung schafft. Da ein zugkraftverstärkendes Mittel eingesetzt wird, welches bei der Anwendung wie z. B. beim Glätten eine höhere Reibungskraft des behandelten bzw. beschichteten Haars schafft, kann bei gleichem Anpressdruck eine erhöhte Zugkraft im Vergleich zu unbehandeltem Haar erzeugt werden. Anders herum kann zum Erzielen der gewünschten Zugkraft, z. B. im Bereich von etwa 2 bis etwa 5 N, die in der Regel für ein gutes Glättergebnis ausreicht, aber noch nicht schmerzhaft oder für die Haare schädlich ist, der Anpressdruck (der auch oft als Normalkraft bezeichnet wird) entsprechend verringert werden. Dadurch wird unter anderem die Gefahr von mechanischen Schädigungen des Haars reduziert. Bei den meisten Haartypen reicht eine Zugkraft von etwa 2 bis 3 N schon für gute Glättergerbnisse aus. Eine Zugkraft in diesem Bereich schafft bei den meisten Haartypen und Glättplattentypen ein ausgewogenes Verhältnis des Glättergebnisses und der Haarschonung. Gleichzeitig werden auch die Glättplatten bzw. deren optionalen Beschichtungen nicht mehr so stark belastet was die Langlebigkeit des Haarglätters fördert. Außerdem sinkt mit sinkendem Anpressdruck auch der Kraftaufwand des Anwenders, so dass die Anwenderfreundlichkeit erhöht wird.

Die Zugkraft hängt zum einen von den Wechselwirkungskräften der Partikel bzw. aktiven Bestandteile gegenüber den Glättplatten und/oder gegenüber der Haaroberfläche und zum anderen von der auf die Haaroberfläche aufgebrachte Menge des Mittels und dessen Verteilung auf der Haaroberfläche ab. Da es sich beim System Haare/Haarwurzel/Kopfhaut um ein natürliches System handelt und diese naturgemäß einer großen Bandbreite bei den Eigenschaften unterliegen, müssen die anzuwenden Zugkräfte beim Haareglätten oder auch beim in Form bringen der Frisur regelmäßig an die individuellen Begebenheiten angepasst werden. Deshalb können die hier angegebenen bevorzugten Bereiche lediglich als Orientierungswerte angesehen werden, die der Fachmann an die jeweilige individuelle Situation durch routinemäßiges Arbeiten anpassen kann. Auch das Schmerzempfinden des Anwenders sowie die verwendete Strähnengröße sind subjektive Parameter, die bei der Anpassung der Zugkraft auf die entsprechende Anwendung zu berücksichtigen sind.

Die vorstehenden vorteilhaften Eigenschaften, insbesondere die Verringerung des im bestimmungsgemäßen Gebrauch erforderlichen Anpressdrucks bei ansonsten gleicher Temperatur bzw. gleichen Glätteigenschaften machen das erfindungsgemäße Mittel geeignet für die Anwendung als Haarstyling- und/oder Haarpflegemittel. Dies gilt insbesondere, wenn es für die Anwendung mit einem beheizbaren Haarglätter ausgestaltet ist.

Bei der Anwendung des erfindungsgemäßen Mittels mit einem Haarglätter können grundsätzlich zwei alternative Ansätze verfolgt werden, um zu berücksichtigen, dass es unterschiedliche Arten von Glättplattenoberflächen gibt. So gibt es zum Beispiel Glättplatten aus Keramikoberflächen, eloxierten Oberflächen, Oberflächen mit einer Emaillegierung oder mit Kautschuken oder Siliconen beschichtete Oberflächen, die alle eine relativ homogene Temperaturverteilung ermöglichen. Jede dieser Oberflächen weist unterschiedliche physikalisch/chemische oder mechanische Haltekräfte (Affinitäten bzw. Reibungskräfte) gegenüber der Haaroberfläche bzw. den erfindungsgemäßen Mitteln auf.

Deshalb kann man das Mittel für die Anwendung als universelles Mittel, das gegenüber mehreren oder allen dieser Glättplattentypen eine zweckmäßige Affinität aufweist, oder als Spezialmittel für einen bestimmten oder ein paar wenige Glättplattentyp(en) ausgestalten.

In der ersten Alternative umfasst das Mittel ein zugkraftverstärkendes Mittel, welches Moleküle und/oder Partikel mit mehreren unterschiedlichen funktionellen Gruppen bzw. eine Mischung daraus umfasst, so dass mehrere oder alle Glättplattenoberflächen mit mindestens einer und bevorzugt zwei oder mehreren funktionellen Gruppen (entweder eines Moleküls oder Partikels oder eines Molekül- oder Partikeltyps) wechselwirken können. Zum Beispiel können Molekülmischungen eingesetzt werden, in denen verschiedene Typen an Molekülen mit jeweils unterschiedlichen funktionellen Gruppen vorhanden sind. Somit kann die Glättplattenoberfläche mit dem Molekültyp wechselwirken, zu dem sie die stärkste Wechselwirkung aufbauen kann, d.h. die beste Affinität zeigt. Ein weiterer Vorteil solcher Molekül- bzw. Partikelmischungen ist, dass die endständigen funktionellen Gruppen nicht nur zu der jeweilig verwendeten Glättplattenoberfläche eine entsprechende Affinität aufweisen müssen, sondern dies auch gegenüber verschiedenen Haaroberflächen tun sollten. So können diese Mittel gleichzeitig mit verschiedenen Haaroberflächen wechselwirken. Die Haaroberfläche, mit der das Mittel in Kontakt kommt, bildet in der Regel die Schuppenschicht (auch Cuticula genannt). Diese Schuppenschicht kann durch Haarpflegemittel, wie z. B. silikonhaltige Schampoos etc., chemisch modifiziert sein. Ein universelles Mittel ist bevorzugt mit funktionellen Gruppen ausgestattet, welche chemische Andockmöglichkeiten an die Schuppenschicht sowie an die Strukturen der gängigsten chemischen Oberflächenmodifizierten Haarpflege- bzw. Haarreinigungsprodukte umfasst. Mit einem solchen universellen Mittel kann auch auf Seiten der Wechselwirkung zu den Haaren eine verbesserte Universallösung gefunden werden, wenn unterschiedliche funktionelle Gruppen in dem Mittel umfasst sind. Beispiele für solche funktionellen Gruppen sind unter anderem Kohlenstoffwasserstoffgruppen, bevorzugt auf Basis langkettiger Kohlenwasserstoffe, oder unpolare siliziumhaltige Gruppen. Beispiele für Moleküle mit solchen funktionellen Gruppen sind z.B. Tenside oder Silane.

Unter eine solche Ausführungsform fällt auch eine zwei- oder mehrstufige Variante, in der in einem ersten Schritt die Haaroberfläche, insbesondere die Cuticula, chemisch modifiziert wird und in einem zweiten Schritt das zugkraftverstärkende Mittel mit dieser chemisch modifizierten Haaroberfläche wechselwirkt. Im ersten Schritt kann zum Beispiel durch die Adsorption einer dünnen, bevorzugt ein oder wenige Moleküllagen dicken Molekülschicht eines Hilfsmittels, wie z. B. eines Schampoos, die Haaroberfläche chemisch modifiziert werden. Diese Molekülschicht kann aufgrund der chemischen Eigenschaften eine zuverlässige Anbindung eines zugkraftverstärkenden Mittels an diese Zwischenschicht gewährleisten. Dadurch kann eine gleichmäßigere Zugkrafteinstellung erzielt werden.

In einer weiteren Ausgestaltung dieser ersten Alternative können die Partikel oder Mischungen daraus auf Grund ihrer Oberflächenstruktur(en), z. B. auf Grund einer mechanischen Wechselwirkung, mit mehreren oder allen Glättplattentypen wechselwirken, so dass man ein universell einsetzbares Mittel erhält.

In der zweiten Alternative ist das erfindungsgemäße Mittel auf einen oder ein paar wenige ähnliche Glättplattentypen ausgelegt und speziell hierfür abgestimmt. In dieser Alternative werden bevorzugt zugkraftverstärkende Mittel mit Molekülen oder Partikeln eingesetzt, die für den jeweiligen Glättplattentyp plattenaffine funktionelle Endgruppen oder auch Mischungen ähnlich wirkender Endgruppen aufweisen. Der Vorteil dieser Mittel ist, dass der Anpressdruck gegenüber den Universalmitteln auf Grund der speziellen Anpassung an den jeweiligen Glättplattentyp geringer gehalten werden kann bzw. eine gegenüber dem universellen Mittel geringere Menge des Mittels benötigt wird, um den gewünschten Effekt der Zugkrafterhöhung bzw. der Anpressdruckverringerung zu erreichen.

Für die schonende Anwendung des Mittels ist es ebenfalls von Vorteil, wenn die Komponenten des Mittels nach der Anwendung, d.h. nach Erfüllung seines Zweckes beim Haarglätten oder in Form bringen der Frisur, von der Haaroberfläche verschwinden. Unter Verschwinden versteht man, dass das Mittel oder dessen Produkte gänzlich oder zumindest zum größten Teil wieder von der Haaroberfläche mechanisch entfernbar sind, z. B. durch Herauskämmen, bzw. es sich während oder kurz nach der Anwendung verflüchtigt bzw. thermisch zersetzt und sich die Zersetzungsprodukte entsprechend entfernen lassen. Die aktiven Komponenten des Mittels unterliegen deshalb bevorzugt einer thermischen Zersetzungsreaktion mit einer geeigneten Kinetik. Läuft die Zersetzungsreaktion zu schnell ab, würden die relevanten aktiven Komponenten (oder zumindest deren aktiven Oberflächenstrukturen) verschwinden, bevor die eigentliche Wechselwirkung zwischen Glättplatten- und Haaroberfläche einsetzt. Verläuft die Reaktion zu langsam, befinden sich nach dem Haareglätten noch Anteile des Mittels im Haar, wodurch gegebenenfalls der Anwender dies als haptisch und/oder optisch oder anderweitig als störend empfinden könnte. Deshalb wird die Zeitspanne für eine solche Zersetzungsreaktion oder anderweitige Verflüchtigung in etwa auf die Dauer des Glättvorgangs abgestimmt.

Typische Mindestzeiträume für solche Anwendungen liegen bei einer oder ein paar wenigen Minuten bis hin zu einer viertel oder halben Stunde. Kommt das Haar mit den aufgeheizten Glättplatten in Kontakt, heizt es sich schnell, d.h. innerhalb etwa 1 s oder ein paar wenigen Sekunden, auf hohe Temperaturen (z.B. ca. 180-200°C) auf und kühlt danach über mehrere Sekunden bis zu mehreren Minuten wieder auf Raumtemperatur ab. Während die Glättplatten und das Haar in Kontakt sind, müssen ausreichend aktive Komponenten des zugkraftverstärkenden Mittels zur Verfügung stehen, um die gewünschten Effekte (z.B. Erhöhung der Zugkraft, Reibungskraft) zu bewirken. Spätestens nach dem Abkühlen der Haare auf Raumtemperatur sind bevorzugt die Komponenten soweit verschwunden, dass sie z.B. haptisch, visuell oder olfaktorisch nicht mehr nachweisbar bzw. zumindest nicht mehr wahrnehmbar sind. Bevorzugt sind die Zersetzungsprodukte gasförmig und verflüchtigen sich leicht. Dabei ist es weiterhin vorteilhaft, dass sie beim Verflüchtigen keine oder nur eine weitgehend nicht wahrnehmbare Geruchsentfaltung zeigen.

Außerdem sind weiter bevorzugt weder die erfindungsgemäßen Mittel noch deren Zersetzungs- oder Abbauprodukte im oder nach dem bestimmungsgemäßen Gebrauch reizend, sonst wie gesundheitsschädlich, entzündlich bzw. explosiv.

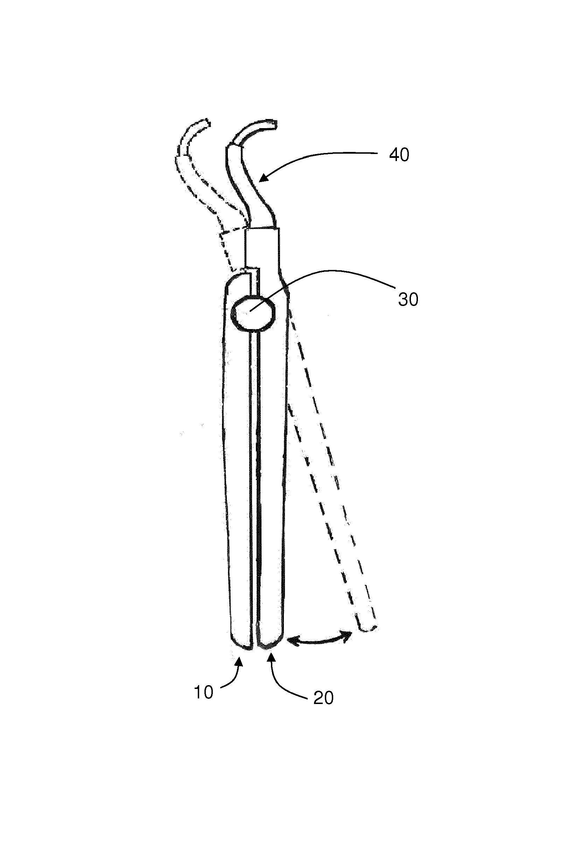

Das Prinzip der Erfindung wird im Folgenden anhand einer Zeichnung beispielshalber noch näher erläutert, wobei die einzige Figur eine Glättvorrichtung für die Anwendung mit einem erfindungsgemäßen Mittel zeigt.

In der Figur ist eine Glättvorrichtung mit einer oberen Glättplatte 10, einer unteren Glättplatte 20, einem Gelenk 30 und einem elektrischen Anschlusskabel 40 im geschlossenen Zustand (durchgezogene Linien) und im offenen Zustand (gestrichelte Linien) gezeigt.

Die beiden Glättplatten 10 und 20 sind über eine innenliegende Widerstandsheizung beheizbar. Das Gelenk 30 verbindet beide Platten derart, dass diese an ihrem vorderen Ende entweder einen schmalen Spalt ausbilden oder in direktem Kontakt kommen. Zwischen die beiden Glättplatten wird bei der Anwendung eine Haarsträhne aus mehreren Haaren gelegt oder eingebracht und durch Zusammenpressen der beiden Glättplatten erwärmt und gehalten. Dadurch dass die Haarsträhne zwischen den Glättplatten mit einem bestimmten Anpressdruck gehalten wird, kann eine Zugkraft auf die Haarsträhne zwischen den Haarwurzeln und dem festgehaltenen Bereich ausgeübt werden. Dadurch wird je nach angewendeter Temperatur und ausgeübter Zugkraft eine Glättung der Haarstruktur erreicht. Dabei sollte darauf geachtet werden, dass kein übermäßiger Anpressdruck auf die Haarsträhne ausgeübt wird, da ansonsten die Gefahr besteht, die Haarstruktur mechanisch zu beschädigen.

### Beispiele

In zwei Beispielen bzw. Studien wurden Gummipartikel als zugkraftverstärkende Elastomerpartikel verwendet, um die Schädlichkeit an Hand von Berechnungen zu simulieren. Schon bei Raumtemperatur oder spätestens bei Wärmezufuhr werden die Partikel verformbar (elastisch), was zu einer Reibungserhöhung zwischen Partikel und Glättplatten führt.

### Beispiel 1

10 µm-Partikel wurden mit einer Häufigkeit von 1 Partikel / 1 mm Haarlänge in der Frisur platziert. Es wurde an Hand dieser Studie kalkuliert, dass bei einer durchschnittlichen Haarlänge von 30 cm und 100.000 Haaren dann etwa 50 mg Partikel im Haar wären. Unter der Annahme, dass sich die Partikel unter Wärme vollständig zersetzt haben, müsste die Raumgröße, in der der Stylingprozess abläuft, mind. 10 m³ betragen, um den so genannten MAK-Wert von 5 mg/m³ für die Zersetzungsprodukte zu erreichen.

Möglicherweise ist bei einer primären Anwendung von privaten Endnutzern auch ein höherer Wert als der MAK-Wert zulässig, denn die Nutzung ist auf ein paar wenige Minuten beschränkt, wobei der MAK-Wert für eine tägliche Aussetzung von acht Stunden ausgeht.

### Beispiel 2

Die Partikelgröße wurde auf 1 µm reduziert und die Partikeldichte auf 100 Partikel / 1 mm Haarlänge erhöht. Die Gesamtmasse der Gummipartikel im Haar wurde so auf ca. 5 mg verringert. Dadurch konnte der MAK-Wert auf alle Fälle eingehalten werden.

Da es sich bei den vorhergehenden, detailliert beschriebenen Mitteln und Verwendungen um Ausführungsbeispiele handelt, können sie in üblicher Weise vom Fachmann in einem weiten Umfang modifiziert werden, ohne den Bereich der Erfindung zu verlassen. Insbesondere können diese Mittel nicht nur zum Glätten der Haare mit einem Haarglätter verwenden werden, sondern sie eignen sich auch für weitere Stylingmethoden wie zum Beispiel Lockenwickeln. Demgemäß ist ein Haarglätter nicht nur ein Gerät mit zwei beheizbaren Platten, sondern kann auch eine Bürste, insbesondere eine beheizbare Bürste, mit einer Haltefunktion für Haarsträhnen sein, mit der ebenso eine Zugkraft unter Erwärmung auf die Haare ausgeübt wird. Auch hier können ähnliche Oberflächenbeschaffenheiten gegeben sein, wie bei einem vorstehend detaillierter beschriebenen Haarglätter. Weiterhin schließt die Verwendung der unbestimmten Artikel "ein" bzw. "eine" nicht aus, dass die betreffenden Merkmale auch mehrfach vorhanden sein können. Auch die Verwendung der Bezeichnungen "Mittel" und "System" schließt nicht aus, dass ein solches Bauteil bzw. ein solches chemisches Mittel aus mehreren Komponenten aufgebaut ist oder noch weitere Bestandteile wie die gewöhnlichen Hilfs- und Zusatzstoffe, wie z.B. Farb- und Geruchsstoffe etc. enthält.

## Patentansprüche

1. Haarstyling- und/oder Haarpflegemittel für die Aufbringung auf das Haar und zur Anwendung im Zusammenwirken mit einem beheizbaren Haarglätter, mit einem bei der Haarglättung zugkraftverstärkenden Mittel.

2. Haarstyling- und/oder Haarpflegemittel nach Anspruch 1, wobei das zugkraftverstärkende Mittel Bestandteile umfasst, deren Moleküle beim bestimmungsgemäßen Gebrauch an der Haaroberfläche adsorbiert werden.

3. Haarstyling- und/oder Haarpflegemittel nach Anspruch 1 oder 2, wobei das zugkraftverstärkende Mittel sich beim bestimmungsgemäßen Gebrauch an der Haaroberfläche anlagernde Partikel umfasst.

4. Haarstyling- und/oder Haarpflegemittel nach einem der vorangehenden Ansprüche, umfassend ein Spreit-Hilfsmittel, welches die Partikel oder Bestandteile nach dem Auftrag auf der Haaroberfläche verteilt.

5. Haarstyling- und/oder Haarpflegemittel nach einem der vorangehenden Ansprüche, wobei das zugkraftverstärkende Mittel Elastomerpartikel umfasst.

6. Haarstyling- und/oder Haarpflegemittel nach einem der vorangehenden Ansprüche, wobei das zugkraftverstärkende Mittel eine alkalische Komponente umfasst.

7. Haarstyling- und/oder Haarpflegemittel nach einem der vorangehenden Ansprüche, wobei das zugkraftverstärkende Mittel eine ölhaltige Komponente umfasst.

8. Haarstyling- und/oder Haarpflegemittel nach einem der vorangehenden Ansprüche, wobei der Partikeldurchmesser der Partikel kleiner oder gleich 200 µm, bevorzugt kleiner oder gleich etwa 150 µm, weiter bevorzugt kleiner oder gleich 100 µm, insbesondere etwa 50 bis 100 µm ist.

9. Haarstyling- und/oder Haarpflegemittel nach einem der vorangehenden Ansprüche, wobei das zugkraftverstärkende Mittel eine Zugkraft zwischen Haaroberfläche und einer Glättplatte einer Glättvorrichtung zwischen 2 bis 5 N, bevorzugt zwischen 2 bis 3 N bei der bestimmungsgemäßen Anwendung schafft.

10. Verwendung eines Haarstyling- und/oder Haarpflegemittels nach einem der vorangehenden Ansprüche für die Anwendung mit einem beheizbaren Haarglätter.
